(19) Europäisches Patentamt
European Patent Office
Office européen des brevets

(11) Veröffentlichungsnummer: **0 321 658 B1**

(12) **EUROPÄISCHE PATENTSCHRIFT**

(45) Veröffentlichungstag der Patentschrift: **16.06.93**

(51) Int. Cl.[5]: **C07C 275/04**, C07C 275/26, C08G 18/78, C08G 18/80

(21) Anmeldenummer: **88115942.0**

(22) Anmeldetag: **28.09.88**

(54) **Verfahren zur Herstellung von blockierten harnstoffgruppenhaltigen Polyisocyanaten sowie die danach hergestellten Produkte.**

(30) Priorität: **21.11.87 DE 3739480**

(43) Veröffentlichungstag der Anmeldung:
**28.06.89 Patentblatt 89/26**

(45) Bekanntmachung des Hinweises auf die Patenterteilung:
**16.06.93 Patentblatt 93/24**

(84) Benannte Vertragsstaaten:
**AT BE CH DE ES FR GB IT LI NL SE**

(56) Entgegenhaltungen:
**DE-A- 2 020 905**
**DE-A- 3 143 060**
**FR-A- 1 233 866**
**US-A- 3 926 875**
**US-A- 4 410 678**

(73) Patentinhaber: **HÜLS AKTIENGESELLSCHAFT**
**Patentabteilung / PB 15 - Postfach 13 20**
**W-4370 Marl 1(DE)**

(72) Erfinder: **Gras, Rainer, Dr.**
**Im Ostholz 49 a**
**W-4630 Bochum 5(DE)**

**EP 0 321 658 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.6/3.3.1)

## Beschreibung

Die Erfindung betrifft eine Herstellung von blockierten harnstoffgruppenhaltigen Polyisocyanaten aus halbblockierten Polyisocyanaten und Polyaminen.

Die Umsetzung von Polyisocyanaten mit Polyaminen erfolgt mit so großer Heftigkeit, daß "sie vor allen anderen Isocyanatreaktionen den Vorzug hat" (vgl. Houben-Weyl, Methoden der organischen Chemie, Band XIII, Seite 132). Diese hohe Reaktivität dürfte auch die Ursache dafür sein, daß die direkte Reaktion zwischen Polyaminen und Polyisocyanaten zu den entsprechenden Harnstoffderivaten lange Zeit wenig untersucht worden ist.

Zwar sind symmetrische Harnstoffderivate im Prinzip auch durch direkte Umsetzung von Polyisocyanaten und Wasser nach der Verfahrensweise der DE-OS 23 41 065 erhältlich, doch muß hierbei in Kauf genommen werden, daß während der Harnstoffbildung gasförmige Reaktionsprodukte entstehen, die sorgfältig entfernt werden müssen. Außerdem haftet dieser Wasser/Isocyanat-Reaktion der Nachteil der begrenzten Variabilität an sowie die Bildung von oligomeren Harnstoffen unter Verbleib von monomerem Polyisocyanat bei stöchiometrischer Verfahrensweise. Zudem lehrt die DE-OS 23 41 065, daß häufig das Polyisocyanat-Isophorondiisocyanat im Überschuß eingesetzt wird bzw. aus Viskositätsgründen Lösemittel verwendet werden müssen.

Ein weiterer Nachteil ist darin zu sehen, daß die hohe Reaktivität der Polyamin/Polyisocyanat-Reaktion nur schwer kontrollierbare Reaktionsabläufe zur Folge hat, was sich in einer breiten Molekulargewichtsverteilung niederschlägt.

Des weiteren wird diese Reaktion auch bei der Herstellung von Biuretpolyisocyanaten beobachtet und stellt dort eine unerwünschte Nebenreaktion dar (vgl. DE-OSS 22 61 065, 26 09 995 und US-PS 3 903 126).

Schließlich wird in der DE-OS 31 43 060 ein Verfahren zur Herstellung von blockierten, Harnstoffgruppen aufweisenden Isophorondiisocyanat-Addukten beschrieben. Solche Verbindungen werden durch Umsetzung von partiell mit Epsilon-Caprolactam blockiertem 3-Isocyanatomethyl-3.5.5-trimethylcyclohexylisocyanat, auch Isophorondiisocyanat (IPDI) genannt, und Diaminen mit zwei sterisch ungehinderten primären und/oder sekundären Aminostickstoffen erhalten. Die Umsetzung erfolgt hierbei in Substanz und muß aus Viskositätsgründen oberhalb 130 °C und, wie in den Beispielen belegt, sogar bei 160 °C erfolgen. Wird die partielle Blockierung von IPDI gemäß der Lehre der DE-OS 31 43 060 durchgeführt, so wird nicht, wie die Reaktionsgleichung aufzeigt und es wünschenswert wäre,

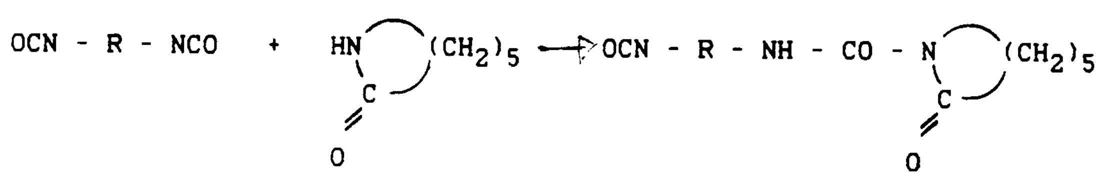

mit R =

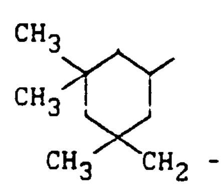

nur das halbblockierte IPDI-Addukt erhalten, sondern es bildet sich unter Erhalt von 15 bis 17 Gew.-% nicht umgesetztem, also unblockiertem monomerem IPDI vollständig blockiertes IPDI. Dieser hohe Monomergehalt stellt einen schwerwiegenden Nachteil dar, da er bei der Umsetzung mit Diaminen für die Bildung unerwünschter polymerer Harnstoffanteile verantwortlich ist.

Für eine gezielte Weiterreaktion kommen die partiell blockierten Polyisocyanate gemäß der Lehre der DE-OS 31 43 060 mit Polyaminen zur Herstellung von für die Polyurethanchemie interessanten Harnstoffverbindungen nicht infrage.

Eine gezielte Weiterreaktion wurde durch die Verwendung eines partiell blockierten Polyisocyanats mit einem Gehalt an monomerem Polyisocyanat bis 6,5 Gew.-%, vorzugsweise bis 3,5 Gew.-% und ganz bevorzugt bis 2,5 Gew.-%, möglich. Die Herstellung dieser partiell blockierten Polyisocyanate erfolgt dadurch, daß man das Polyisocyanat im großen Überschuß - 5 bis 20 Mol - mit dem Blockierungsmittel - 1

Mol - bei Temperaturen von 50 °C bis 130 °C, vorzugsweise 70 °C bis 100 °C, zur Reaktion bringt und anschließend das überschüssige Polyisocyanat durch Dünnschichtdestillation abtrennt. Diese Herstellung ist Gegenstand einer gesonderten Patentanmeldung.

Gegenstand der Erfindung ist daher ein Verfahren zur Herstellung von blockierten harnstoffgruppenhaltigen Polyisocyanaten aus halbblockierten Polyisocyanaten und Polyaminen, dadurch gekennzeichnet, daß man ein mittels Dünnschichtdestillation weitgehend von unblockiertem, monomerem Polyisocyanat befreites halbblockiertes Polyisocyanat mit primären und/oder sekundären Polyaminen im Isocyanat-/Aminogruppen-Verhältnis von 1 bis 1,3 : 1, bevorzugt 1 : 1 bei einer Temperatur im Bereich von RT bis 80 °C umsetzt, wobei das zur Dünnschichtdestillation eingesetzte Polyisocyanat einen Siedepunkt im Vakuum unterhalb der Deblockierungstemperatur des eingesetzten Blockierungsmittels besitzt, das halbblockierte Polyisocyanat durch Zugabe von 1 Mol Blockierungsmittel zu 5 bis 20 Mol Polyisocyanat erhalten wird und das halbblockierte Polyisocyanat nach der Dünnschichtdestillation einen Gehalt an monomerem, unblockiertem Polyisocyanat bis 6,5 Gew.-%, vorzugsweise bis 3,5 Gew.-% und insbesondere bis 2,5 Gew.-%, aufweist.

Als Polyamine werden solche gemäß den Formel I und II verwendet

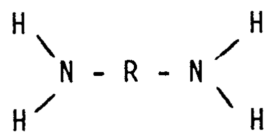

I

worin

R = $(CH_2)$-$_{2-36}$, geradkettig oder alkylverzweigt,

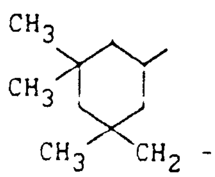
,
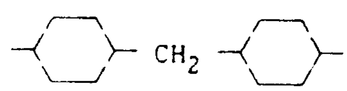
,

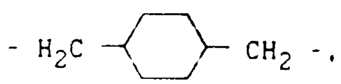
,
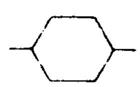
,
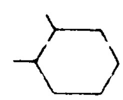
,

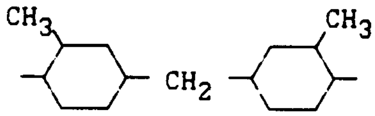 , 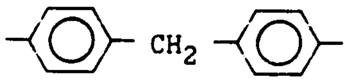 , 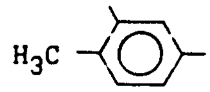 ,

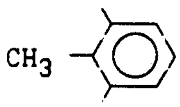

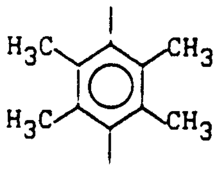

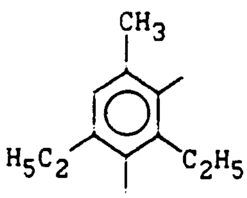 , 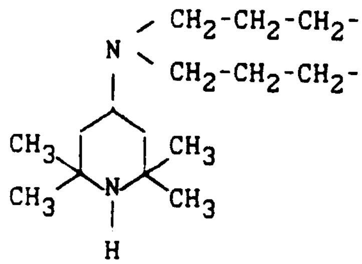

und

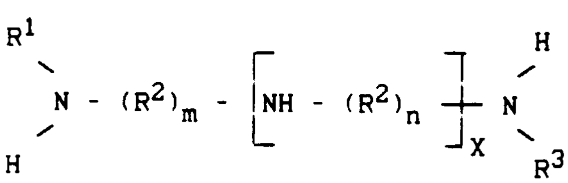 II

worin

- $R^1$ = gleich oder verschieden von $R^3$ = H, alkyl-, (cyclo)-alkyl-, aryl-, aralkyl, ggf. alkylverzweigt
- $R^2$ = $CH_2$-, ggf. alkylverzweigt
- m = 2 bis 8
- X = 0 bis 6
- n = 2 bis 8

ist.

Die erfindungsgemäß einzusetzenden Polyisocyanate sind z. B. in "Methoden der organischen Chemie, Houben-Weyl, Bd. 14/2, 4. Auflage, Georg Thieme Verlag Stuttgart, 1963, S. 61-70 und bei W. Siefken, Liebigs Ann. Chem. 562, S. 75-136, beschrieben. Es kommen nur solche Polyisocyanate infrage, deren Siedepunkte im Vakuum unterhalb der Deblockierungstemperatur des eingesetzten Blockierungsmittels liegen und somit mittels Dünnschichtdestillation abgetrennt werden können.

Besonders bevorzugt werden die technisch leicht zugänglichen aliphatischen, (cyclo)aliphatischen und aromatischen Polyisocyanate wie Hexamethylendiisocyanat-1.6 (HDI), 2.2.4(2.4.4)-Trimethylhexamethylendiisocyanat (TMDI), 2-Methylpentandiisocyanat-1.5 (DI51), und 2.4- bzw. 2.6-Toluylendiisocyanat, insbesondere das 3-Isocyanatomethyl-3.5.5-trimethylcyclohexylisocyanat (IPDI) sowie deren Isomerengemische.

Für die Herstellung der erfindungsgemäßen Polyisocyanat-Harnstoff-Addukte kommen Polyamine mit primären und/oder sekundären Aminogruppen infrage. Sie enthalten 2 bis 36 C-Atome und können aliphatischer Art sein oder einen oder mehrere cycloaliphatische, aromatische oder heterocyclische 5- bis 8-gliedrige Ringe enthalten. Zusätzlich können die einsetzbaren Polyamine auch tertiäre Aminogruppen tragen.

Beispielhaft seien genannt:

- unverzweigte primäre Alkylendiamine, wie Ethylendiamin, Trimethylendiamin, Tetramethylendiamin, Hexamethylendiamin, Octamethylendiamin, Dodecamethylendiamin und das $C_{36}$-Diamin,
- verzweigte primäre Alkylendiamine, wie 2-Methyl-pentamethylendiamin, 2.2.4(2.4.4)-Trimethylhexamethylendiamin und 5-Methyl-nonamethylendiamin,
- cycloaliphatische primäre Diamine wie 1.4(1.2)-Diaminocyclohexan, 2.4(2.6)-Diamino-1-methylcyclohexan, 4.4'-Diaminodicyclohexylmethan, 4.4'-Diamino-3.3'-dimethyldicyclohexylmethan, 4.4'-Diamino-3.3'5.5'-tetramethyldicyclohexylmethan,
- cycloaliphatische/aliphatische primäre oder sekundäre Diamine, wie N-Cyclohexyl-propylendiamin-1.3, Bis-(1.4-aminomethyl)-cyclohexan und 3-Aminomethyl-3.5.5-trimethylcyclohexylamin (Isophorondiamin - IPD),
- aliphatische primäre und sekundäre Polyamine wie Diethylentriamin, Triethylentetramin, Tetraethylenpentamin, Pentaethylenhexamin, Dipropylentriamin, Dihexamethylentriamin, Dioctamethylentriamin und Bis-(3-aminopropyl)-ethylendiamin,
- aromatische primäre Diamine wie 3.5-Diethyl-toluylendiamin, 2.4(2.6)-Toluylendiamin, 1.4-Diamino-2.3.5.6-tetramethylbenzol und 4.4'-Diaminodiphenylmethan,
- aliphatische sekundäre Diamine wie 1.1.6.6-Tetraisopropyl-2.5-diazahexan und N.N'-Diisobutylisophorondiamin,
- heterocyclische Diamine wie 4(3-Aminopropyl-amino)-2.2.6.6-tetramethylpiperidin und 4[(Bis-3-aminopropyl)amino]-2.2.6.6-tetramethylpiperidin,
- sowie deren Gemische.

Es können auch Melamin und Wasser verwendet werden.

Der Einsatz der genannten heterocyclischen Verbindungen ist von großem Interesse, da sie es gestatten, permanente UV-stabilisierte Kunststoffe, insbesondere auch Lacksysteme, herzustellen. Sie fungieren hierbei nicht nur einfach als Additiv, sondern sie sind direkt in das Makromolekül eingebaut.

Als Blockierungsmittel kommen erfindungsgemäß solche infrage, die bei Härtungsbedingungen, d. h. zwischen 140 und 220 °C, respalten. Dazu werden Alkohole, z. B. Methanol, Ethanol, Isopropanol, Cyclohexanol, Oxime, wie Acetonoxim, Methylisobutylketoxim, Mercaptane, Lactame, z. B. Lauryllactam, Acetessigester, Malonsäureester und insbesondere Epsilon-Caprolactam und Methylethylketoxim gezählt (vgl. "Methoden der Organischen Chemie", Houben-Weyl, Bd. 14/2, 4. Aufl., Georg Thieme Verlag Stuttgart, 1963, S. 61 ff.).

Die Herstellung der Härter erfolgt erfindungsgemäß im Lösungsmittel und zwar so, daß das partiell blockierte Polyisocyanat im Lösungsmittel vorgelegt wird und das Polyamin entweder in Substanz oder ebenfalls im Lösungsmittel gelöst zugetropft wird. Geeignete Lösungsmittel sind einmal solche, die leicht entfernt werden können, wie Toluol, Xylol, Cyclohexan, Spezialbenzin (mit = 1 Vol.-% Aromaten), Essigester und Aceton, um die erfindungsgemäßen Polyisocyanat- Harnstoff-Addukte zu isolieren, zum anderen solche Lösungsmittel wie Toluol, Xylol, SOLVESSO® 100 und 150 (Aromatengemische der Fa. Esso), Tetralin, Cumol, Methylisobutyl- und Diisobutylketon, Essigsäurehexyl-, Essigsäurebutylester, Ethylglykolacetat (EGA), Methoxypropylacetat (MOP-acetat), Butylglykolacetat usw., die in praxi für lösungsmittelhaltige Lacksysteme eingesetzt werden.

Die genannten Verbindungen können auch als Gemische verwendet werden. Die Konzentrationen der Polyisocyanat-Harnstoff-Addukte können in weiten Grenzen schwanken. Als vorteilhaft haben sich Konzentrationen von 30 bis 70 Gew.-% erwiesen.

Die Umsetzung findet erfindungsgemäß zwischen RT und 80 °C, vorzugsweise zwischen RT und 70 °C statt. Nach erfolgter Polyaminzugabe ist es vorteilhaft, daß das Reaktionsgemisch auf gegebenenfalls 100 bis 120 °C erhitzt wird. Soll das Reaktionsprodukt vom Lösungsmittel befreit werden, so kann das durch einfaches Anlegen von Vakuum erfolgen. Besonders geeignet zur Beseitigung des Lösungsmittels ist die Schmelzextrusion in einer Abdampfschnecke.

Als besonders vorteilhaftes Verfahren zur erfindungsgemäßen Herstellung der festen, blockierten Polyisocyanat-Harnstoff-Addukte hat sich die Verwendung von Spezialbenzinen mit = 1 Vol.-% Aromaten oder Cyclohexan als Lösungsmittel erwiesen. Die Reaktion wird hier bei Raumtemperatur durchgeführt, und zwar so, daß das halbblockierte Polyisocyanat in Spezialbenzin oder Cyclohexan gelöst wird und das Polyamin unter intensivem Rühren so zugetropft wird, daß die Reaktionstemperatur 70 °C nicht übersteigt. Das Reaktionsprodukt fällt während des Zutropfvorganges aus. Nach beendeter Aminzugabe wird noch 30 bis 45 Minuten weiter gerührt und anschließend vom Lösungsmittel befreit.

Bei der erfindungsgemäßen Umsetzung werden die Reaktionspartner - Polyisocyanat und Polyamin - im Isocyanat/Aminogruppen-Verhältnis von 1-1,3:1, vorzugsweise pro Isocyanatgruppe eine primäre und/oder sekundäre Aminogruppe eingesetzt. Die erfindungsgemäßen Addukte besitzen im allgemeinen einen NCO-Gehalt von 4 bis 20, vorzugsweise von 6 bis 15 Gew.-%. Der freie Isocyanatgehalt beträgt = 0,7 Gew.-%

bis 5 Gew.-%. Die Polyisocyanatadditionsprodukte weisen einen Schmelzbereich von 90 bis 210 °C auf. Sie eignen sich insbesondere als Härter für Zerewitinoff-aktive Wasserstoffatome aufweisende höherfunktionelle Verbindungen. In Kombination mit derartigen Zerewitinoff-aktiven Wasserstoffatome aufweisenden Verbindungen führen die Polyadditionsprodukte oberhalb 140 °C, vorzugsweise 180 bis 220 °C zu hochwertigen Kunststoffen aushärtenden Systemen. Das sicherlich bedeutendste Anwendungsgebiet für derartige Systeme ist ihre Verwendung als Vernetzer für PUR-Pulverlacke sowie lösungsmittelhaltige Einkomponenten-PUR-Einbrennlacke.

Die erfindungsgemäßen Verbindungen finden als Zwischenprodukte zur Herstellung von Kunststoffen, insbesondere von Lacken, Verwendung. Sie sind deshalb besonders wertvoll, weil sie es gestatten, auf einfache Weise auch PUR-Pulverlacke mit reduziertem Glanzgrad herzustellen.

## Experimenteller Teil

### A Herstellung der partiell blockierten Polyisocyanate

Allgemeine Herstellungsvorschrift

Zu 5 bis 20 Mol Polyisocyanat wird bei 70 bis 80 °C unter Rühren portionsweise 1 Mol Blockierungsmittel zugegeben. Nach beendeter Blockierungsmittelzugabe wird das Reaktionsgemisch noch eine Stunde bei 100 °C erhitzt und anschließend das nicht umgesetzte Polyisocyanat mittels Dünnschichtdestillation bei 90 bis 140 °C und 0,0133 mbar entfernt. Vom Reaktionsprodukt (= Rückstand) wurden die chemischen und physikalischen Kenndaten bestimmt und in der folgenden Tabelle zusammengefaßt.

Partiell blockierte Polyisocyanate

| Beispiele | Ausgangsstoffe | | NCO-Gehalt in Gew.-% | | Polyisocyanat monomer | Viskosität in mPa·s bei °C | | | | |
|---|---|---|---|---|---|---|---|---|---|---|
| A | Polyiso-cyanat | Blockierungs-mittel | frei | gesamt | Gew.-% | 25 | 30 | 40 | 50 | 70 |
| 1 | IPDI | Caprolactam | 12,4 | 24,8 | < 1,0 | 1250000 | 410000 | 63500 | 13700 | 1250 |
| 2 | IPDI | Caprolactam | 11,5 | 24,5 | 2,2 | 1145000 | 397000 | 61250 | 12530 | 1070 |
| 3 | IPDI | Caprolactam | 11,6 | 24,4 | 3,5 | 1120000 | 360000 | 59500 | 11500 | 980 |
| 4 | IPDI | Caprolactam | 11,8 | 24,5 | 4,6 | 660000 | 194000 | 34000 | 8550 | 680 |
| 5 | IPDI | Caprolactam | 11,85 | 24,6 | 6,4 | 451000 | 101000 | 22000 | 4500 | 450 |
| 6 | IPDI | MEK-oxim | 12,8 | 26,5 | 2,8 | 571000 | 152000 | 27500 | 5500 | 420 |
| 7 | HDI | Caprolactam | 15,3 | 29,4 | 0,5 | 100 | 80 | 50 | 30 | < 30 |
| 8 | HDI | MEK-oxim | 15,8 | 32,4 | 2,5 | 70 | 60 | 50 | 30 | < 30 |
| 9 | DI51 | Caprolactam | 14,8 | 29,3 | 0,7 | 160 | 120 | 60 | 45 | < 30 |
| 10 | DI51 | MEK-oxim | 15,7 | 31,5 | 0,8 | 190 | 150 | 80 | 60 | 30 |
| 11 | DI51 | MEK-oxim | 16,5 | 32,9 | 7,5 | 110 | 70 | 40 | < 30 | < 30 |

B Herstellung der erfindungsgemäßen blockierten Polyisocyanat-Harnstoff-Addukte

Zu einer 50 bis 70 %igen toluolischen Lösung eines partiell blockierten Polyisocyanates werden - bezogen auf den freien NCO-Gehalt - die äquivalenten Mengen einer 50 bis 70 %igen toluolischen Lösung

eines Diamins oder Polyamins oder deren Gemische bei 55 bis 65 °C unter intensivem Rühren so zugegeben, daß die Reaktionstemperatur 70 °C nicht übersteigt. Nach beendeter Aminzugabe wird das Reaktionsgemisch noch 30 bis 45 Minuten weiter erhitzt. Während dieser Zeit wird die Temperatur auf 100 bis 120 °C langsam erhöht. Anschließend wird das Reaktionsprodukt im Vakuum bei 0,133 mbar vom Toluol befreit. In den folgenden Tabellen sind die chemischen und physikalischen Kenndaten der Reaktionsprodukte zusammengefaßt:

a) IPDI-Harnstoff-Addukte gemäß A 2 und Aminkomponente
b) IPDI-Harnstoff-Addukte gemäß A 1 und Aminkomponente
c) HDI-Harnstoff-Addukt gemäß A 7 und Aminkomponente
d) DI51-Harnstoff-Addukt gemäß A 9 und Aminkomponente
e) IPDI-Harnstoff-Addukte gemäß A 3, A 4, A 5 und Aminkomponente
f) IPDI-Harnstoff-Addukte gemäß A 2 im $NCO/NH_2$-Verhältnis von 1,05 bis 1,3:1 und Aminkomponente
g) IPDI-Harnstoff-Addukte gemäß A 2 und Aminkomponente, hergestellt zwischen 40 und 110 °C
h) IPDI-Harnstoff-Addukte gemäß A 2 und Aminkomponente, hergestellt in Aceton oder Essigester
i) IPDI-Harnstoff-Addukte gemäß A 2 und Wasser

j) IPDI-Harnstoff-Addukte gemäß A 2 und Aminkomponente, hergestellt in Spezialbenzin oder Cyclohexan

| Beispiel B a | Amin-Komponente | NCO-Gehalt (Gew.-%) | | Schmelzbereich | Glasumwandlungstemperatur |
|---|---|---|---|---|---|
| | | latent | frei | °C | (DTA) °C |
| 1 | Hexamethylendiamin-1.6 | 10,8 | 0.15 | 120-122 | 51-66 |
| 2 | Octamethylendiamin-1.8 | 10,5 | 0,5 | 101-103 | 41-61 |
| 3 | Dodecamethylendiamin-1.12 | 9,6 | 0,3 | 93- 96 | 32-56 |
| 4 | 2-Methyl-pentamethylen-diamin-1.5 | 10,5 | 0 | 106-108 | 35-47 |
| 5 | 2.2.4(2.4.4)-Trimethyl-hexamethylendiamin-1.6 (TMD) | 10,0 | 0 | 107-109 | 30-44 |
| 6 | 1.4-Diaminocyclohexan | 10,3 | 0,3 | 120-130 | 53-85 |
| 7 | 4.4'-Diamino-dicyclohexyl-methan - fest | 9,9 | 0,25 | 148-150 | 39-58 |
| 8 | 4.4'-Diamino-dicyclohexyl-methan - flüssig | 10,0 | 0,3 | 146-149 | 40-57 |
| 9 | 3.3'-Dimethyl-4.4'-diamino-dicyclohexylmethan | 9,3 | 0 | 149-155 | 37-85 |
| 10 | N-Cyclohexyl-propylen-diamin-1.3 | 10,8 | 0,3 | 142-145 | 43-80 |
| 11 | Bis-(1.4-aminomethyl)-cyclohexan | 10,9 | 0,5 | 133-140 | 50-80 |
| 12 | Isophorondiamin (IPD) | 10,5 | 0,1 | 160-165 | 60-87 |

| Beispiel B a | Amin-Komponente | NCO-Gehalt (Gew.-%) latent | NCO-Gehalt (Gew.-%) frei | Schmelzbereich °C | Glasumwandlungstemperatur (DTA) °C |
|---|---|---|---|---|---|
| 13 | 90 Gew.-T. IPD<br>10 Gew.-T. TMD | 9,9 | 0 | 146-157 | 49-72 |
| 14 | 70 Gew.-T. IPD<br>30 Gew.-T. TMD | 9,9 | 0 | 138-148 | 38-61 |
| 15 | 50 Gew.-T. IPD<br>50 Gew.-T. TMD | 10,2 | 0,1 | 125-127 | 34-58 |
| 16 | Diethylentriamin | 11,1 | 0,4 | 162-165 | 45-88 |
| 17 | Triethylentetramin | 11,5 | 0,5 | 140-145 | 40-80 |
| 18 | Tetraethylenpentamin | 12,0 | 0,3 | 120-123 | 40-75 |
| 19 | Pentamethylenhexamin | 12,1 | 0,4 | 113-115 | 36-70 |
| 20 | Dipropylentriamin | 11,8 | 0,4 | 153-155 | 40-65 |
| 21 | Bis-(3-aminopropyl)-ethylendiamin | 11,6 | 0,5 | 152-155 | 42-68 |
| 22 | 3.5-Diethyl-toluylen-diamin | 10 | 0 | 203-205 | 70-80 |
| 23 | 1.4-Diamino-2.3.5.6-tetramethylbenzol | 10 | 0 | 175-177 | 55-72 |
| 24 | 4.4'-Diaminodiphenyl-methan | 10 | 0,3 | 178-182 | 58-74 |

| Beispiel B a | Amin-Komponente | NCO-Gehalt (Gew.-%) latent | frei | Schmelzbereich °C | Glasumwandlungstemperatur (DTA) °C |
|---|---|---|---|---|---|
| 25 | 1.1.6.6-Tetraisopropyl-2.5-diazahexan | 9,7 | 0,45 | 120-122 | 47-65 |
| 26 | N.N'-Diisobutyl-isophorondiamin | 9,4 | 0,3 | 130-132 | 45-65 |
| 27 | 4(3-Aminopropyl-amino)-2.2.6.6-tetramethylpiperidin | 10,1 | 0,5 | 125-129 | 35-49 |
| 28 | 4[(Bis-3-aminopropyl)-amino]-2.2.6.6-tetramethylpiperidin | 9,6 | 0,5 | 138-140 | 40-53 |
| 29 | Melamin | 10,3 | 0,5 | 197-200 | 75-90 |
| 30 | N-(3-Aminopropyl)glucamin | 9,5 | 0 | 124-126 | 33-95 |
| Beispiel B b | | | | | |
| 1 | 4.4'-Diamino-dicyclohexylmethan - flüssig | 10,2 | 0,1 | 144-147 | 41-59 |
| 2 | Bis-(1.4-aminomethyl)cyclohexan | 10,8 | 0,3 | 136-141 | 53-75 |
| Beispiel B c | | | | | |
| 1 | 4.4'-Diaminodicyclohexylmethan - flüssig | 10,7 | 0 | 124-126 | 26-52 |
| Beispiel B d | | | | | |
| 1 | 4.4'-Diaminodicyclohexylmethan - flüssig | 10,6 | 0,1 | 105-111 | 24-52 |

Beispiel B e

Gemäß der allgemeinen Herstellungsvorschrift B wurden die partiell blockierten IPDI-Addukte gemäß A 3, A 4 und A 5 mit 4.4'-Diaminodicyclohexylmethan zur Reaktion gebracht. In der folgenden Tabelle sind die chemischen und physikalischen Kenndaten dargestellt:

| Beispiel B e | IPDI-Add. gemäß | NCO-Gehalt in Gew.-% | | Schmelzbereich °C | Glasumwandlungstemperatur (DTA) °C |
|---|---|---|---|---|---|
| | | latent | frei | | |
| 1 | A 3 | 10,3 | 0,3 | 148-150 | 38-72 |
| 2 | A 4 | 10,2 | 0,1 | 155-161 | 37-78 |
| 3 | A 5 | 10,25 | 0,2 | 155-157 | 40-96 |

Beispiel B f

Gemäß der allgemeinen Herstellungsvorschrift B wurde partiell blockiertes IPDI gemäß A 2 mit 4.4′-Diaminodicyclohexylmethan nicht im äquivalenten Verhältnis, sondern im NCO/Amin-Verhältnis = X : 1 umgesetzt. In der folgenen Tabelle sind die chemischen und physikalischen Kenndaten zusammengefaßt:

| Beispiel B f | X | NCO-Gehalt in Gew.-% | | Schmelzbereich °C | Glasumwandlungstemperatur (DTA) °C |
|---|---|---|---|---|---|
| | | gesamt | frei | | |
| 1 | 1,05 | 9,9 | 0,4 | 145-148 | 35-58 |
| 2 | 1,1 | 10,7 | 0,8 | 140-142 | 33-59 |
| 3 | 1,2 | 11,5 | 1,4 | 138-140 | 35-58 |
| 4 | 1,3 | 12,0 | 1,95 | 131-136 | 33-55 |

Beispiel B g

Gemäß der allgemeinen Herstellungsvorschrift für die blockierten Polyisocyanat-Harnstoff-Addukte wurde bei X °C partiell blockiertes IPDI gemäß Beispiel A 2 mit 4.4'-Diaminodicyclohexylmethan zur Reaktion gebracht und in der folgenden Tabelle die chemischen und physikalischen Kenndaten der erfinderischen und nichterfinderischen Reaktionsprodukte gegenübergestellt:

| Beispiele B g | | Reaktionstemperatur X °C | NCO-Gehalt in Gew.-% | | Glasumwandlungstemperatur in °C (DTA) |
|---|---|---|---|---|---|
| | | | latent | frei | |
| 1 | **I** | 40 | 10,27 | 0,2 | 36- 59 |
| 2 | | 50 | 10,25 | 0,3 | 38- 58 |
| 3 | | 60 | 10,1 | 0,2 | 40- 57 |
| 4 | | 70 | 10,3 | 0,25 | 40- 59 |
| 5 | | 80 | 10,0 | 0,15 | 40- 56 |
| 6 | **II** | 90 | 10,3 | 0,1 | 28- 81 |
| 7 | | 100 | 10,2 | 0 | 19- 90 |
| 8 | | 110 | 10,15 | 0,2 | 17-125 |
| Bemerkungen:<br>**I** = Erfindung<br>**II** = Vergleich | | | | | |

Beispiel B h1

Zu 730 Gew.-T. partiell blockiertem IPDI gemäß A 2, gelöst in 506 Gew.-T. Aceton, werden - bezogen auf den freien NCO-Gehalt - 210 Gew.-T. 4.4'-Diamino-dicyclohexylmethan bei 55 bis 60 °C unter intensivem Rühren so zugegeben, daß die Reaktionstemperatur 65 °C nicht übersteigt. Nach beendeter

Diaminzugabe wird das Reaktionsgemisch noch 30 bis 45 Minuten weiter gerührt, bis der NCO-Gehalt auf = 0,5 Gew.-% gesunken ist. Anschließend wird das Reaktionsprodukt im Vakuum bei 0,133 mbar vom Aceton befreit. Das Reaktionsprodukt hatte folgende chemischen und physikalischen Kenndaten:

| | |
|---|---|
| NCO-Gehalt (latent) | 10,3 Gew.-% |
| NCO-Gehalt (frei) | 0,3 Gew.-% |
| Schmelzbereich | 147-149 °C |
| Glasumwandlungstemperatur (DTA) | 40- 59 °C |

Beispiel B h2

Gemäß Beispiel B h1 wird die Reaktion im Essigester ausgeführt. Das Reaktionsprodukt hatte folgende chemischen und physikalischen Kenndaten:

| | |
|---|---|
| NCO-Gehalt (latent) | 10,04 Gew.-% |
| NCO-Gehalt (frei) | 0 Gew.-% |
| Schmelzbereich | 146-148 °C |
| Glasumwandlungstemperatur (DTA): | 46- 59 °C |

Beispiel B i

Zu X Gew.-T. partiell blockiertem IPDI gemäß Beispiel A 2 und 0,15 Gew.-% DBTL, bezogen auf eingesetztes partiell blockiertes IPDI, gelöst in Y Gew.-T. Aceton, werden - bezogen auf den freien NCO-Gehalt - Z Gew.-T. Wasser bei 50 bis 60 °C unter intensivem Rühren so zugegeben, daß die Reaktionstemperatur 65 °C nicht übersteigt. Nach beendeter Wasserzugabe wird noch so lange unter Rühren weiter erhitzt, bis die berechnete Menge $CO_2$ abgespalten worden ist. Anschließend wird die Umsetzung mittels titrimetrischer NCO-Bestimmung kontrolliert und danach das Reaktionsprodukt im Vakuum bei 0,133 mbar vom Aceton befreit. In der nachstehenden Tabelle sind die Rezepturen sowie die entsprechenden chemischen und physikalischen Kenndaten zusammengefaßt:

| Beispiel B i | X | Y | Z | $CO_2$ in l | NCO-Gehalt in Gew.-% | | Schmelzbereich °C | DTA * °C |
|---|---|---|---|---|---|---|---|---|
| | | | | | gesamt | frei | | |
| 1 | 359 | 146 | 9 | 11,3-12 | 12,5 | 0,3 | 105-107 | 45-65 |
| 2 | 185 | 120 | 3,45 | 4,3-4,6 | 15,2 | 2,1 | 94- 96 | 38-56 |

* Glasumwandlungstemperatur

Beispiel B j

Zu 730 Gew.-T. partiell blockiertem IPDI gemäß Beispiel A 2, gelöst in 500 bis 550 Gew.-T. Spezialbenzin (enthält 3 Vol-% Aromaten) oder Cyclohexan wird - bezogen auf den freien NCO-Gehalt - die berechnete Menge Aminkomponente bei Raumtemperatur unter heftigem Rühren so zugetropft, daß die Reaktionstemperatur 70 °C nicht übersteigt. Das Polyisocyanat-Harnstoff-Addukt fällt bereits während der Diaminzugabe aus. Nach beendeter Diaminzugabe wird noch 30 bis 45 Minuten weiter gerührt und anschließend, nach der titrimetrischen NCO-Bestimmung, vom Lösungsmittel befreit. Die folgende Tabelle gibt die chemischen und physikalischen Kenndaten wieder:

| NCO/Amin-Verhältnis = 1 : 1 | | | | | |
|---|---|---|---|---|---|
| Beispiel B j | Aminkomponente | NCO-Gehalt in Gew.-% | | Schmelz-bereich | Glasumwand-lungstemperatur |
| | | gesamt | frei | °C | (DTA) °C |
| 1 | 4.4'-Diaminodi-cyclohexylmethan - flüssig | 9,5 | 0,4 | 146-149 | 36-63 |
| 2 | Bis-(1.4-aminome-thyl-cyclohexan | 10,2 | 0,4 | 134-136 | 45-95 |
| 3 | Isophorondiamin | 10,0 | 0,4 | 154-159 | 56-84 |
| 4 | Pentaethylen-hexamin | 11,3 | 0 | 123-127 | 37-65 |

| NCO/Amin-Verhältnis 1,1 : 1,2 : 1,3 : 1 | | | | | |
|---|---|---|---|---|---|
| Beispiel B j | Aminkomponente | NCO-Gehalt in Gew.-% | | Schmelz-bereich | Glasumwand-lungstemperatur |
| | | gesamt | frei | °C | (DTA) °C |
| 5 | 4.4'Diaminodi-cyclohexylmethan | 10,9 | 0,8 | 137-141 | 37-60 |
| 6 | " | 11,2 | 1,5 | 140-141 | 33-59 |
| 7 | " | 12,1 | 1,9 | 133-135 | 32-56 |

C Herstellung der erfindungsgemäßen blockierten Polyisocyanat-Harnstoff-Addukte in lacküblichen Lösungsmitteln

Allgemeine Herstellungsvorschrift

Zu einer 30 bis 50 %igen lacküblichen Lösemittellösung eines partiell blockierten Polysiocyanats werden - bezogen auf den freien NCO-Gehalt - die äquivalenten Mengen - gelöst oder in Substanz - eines Diamins oder Polyamins oder deren Gemische bei 50 bis 60 °C unter intensivem Rühren so zugegeben, daß die Reaktionstemperatur 70 °C nicht übersteigt. Nach beendeter Aminzugabe wird das Reaktionsgemisch noch so lange weiter erhitzt, bis der NCO-Gehalt auf Null gesunken ist. In der folgenden Tabelle sind die chemischen und physikalischen Kenndaten zusammengefaßt:

| Beispiel C a | Aminkomponente | Konzentration des Harnstoff-Adduktes | Lösemittel | NCO-Gehalt (latent) Gew.-% | Viskosität DIN-4-Becher 20 °C in s | mPa·s 25 °C |
|---|---|---|---|---|---|---|
| 1 | 2.2.4(2.4.4)-Trimethylhexa-methylendiamin-1.6 (TMD) | 40 | S* 100 | 4,2 | 116 | 330 |
| | | 50 | E*/S 100 1:2 | 5,3 | 345 | 1 040 |
| 2 | Pentaethylenhexamin (PEHA) | 40 | S 100 | 4,6 | 32 | 90 |
| | | 50 | E/S 100 1:2 | 5,8 | 200 | 644 |
| 3 | Bis-(1.4-aminomethyl)-cyclo-hexan (HXDA) | 40 | E/S 100 1:2 | 4,3 | - | 7 000 |
| 4 | Isophorondiamin (IPD) | 30 | E/S 100 1:2 | 3,1 | 151 | 410 |
| | | 40 | E/S 100 1:2 | 4,1 | - | 15 600 |
| 5 | HXDA/PEHA 2 : 1 | 30 | E/S 100 1:2 | 3,4 | 190 | 489 |
| | | 40 | E/S 100 1:2 | 4,2 | 190 | 490 |
| 6 | IPD/PEHA 2 : 1 | 40 | E/S 100 1:2 | 4,2 | 166 | 472 |
| 7 | HXDA/TMD 1 : 1 | 30 | E/S 100 1:2 | 3,1 | 72 | 177 |
| | | 40 | E/S 100 1:2 | 4,1 | 754 | 2 086 |

S* = SOLVESSO®, E* = EGA

Beispiel C b

Zu X Gew.-T. partiell blockiertem IPDI gemäß Beispiel A 1 und 0,15 Gew.-% DBTL, bezogen auf eingesetztes partiell blockiertes IPDI, gelöst in Y Gew.-T. lacküblichem Lösungsmittel, werden - bezogen auf den freien NCO-Gehalt - Z Gew.-T. Wasser bei 55 bis 60 °C unter intensivem Rühren so zugegeben,

daß die Reaktionstemperatur 65 °C nicht übersteigt. Nach beendeter Wasserzugabe wird noch so lange unter Rühren weiter erhitzt, bis die berechnete Menge $CO_2$ abgespalten worden ist. Anschließend wird die Umsetzung mittels titrimetrischer NCO-Bestimmung kontrolliert. In der folgenden Tabelle sind die Rezepturen und die entsprechenden chemischen und physikalischen Kenndaten der Vernetzerlösungen angegeben:

| Beispiel C b | X | Y | Z | $CO_2$ in l | NCO-Gehalt in Gew.-% | | Auslaufzeit im DIN-4-Becher 20 °C in s |
|---|---|---|---|---|---|---|---|
| | | | | | gesamt | frei | |
| 1 | 185 | 120 | 3,45 | 4,2-4,6 | 9,4 | 1,6 | 215 |
| 2 | 183,3 | 120 | 2,3 | 2,8-3,0 | 10,9 | 3,4 | 50 |
| 3 | 181,6 | 120 | 1,15 | 4,2-4,5 | 12,8 | 5,0 | 20 |

D Vergleichsbeispiele

I Allgemeine Herstellungsvorschrift (Partiell blockierte Polyisocyanate ohne Dünnschichtdestillation)

Zu einem Mol Polyisocyanat wird bei 90 bis 110 °C ein Mol Blockierungsmittel so zugegeben, daß die Temperatur des Reaktionsgemisches nicht über 120 °C ansteigt. Nach beendeter Blockierungsmittelzugabe wird noch so lange erhitzt, bis der NCO-Gehalt des Reaktionsgemisches den berechneten Wert erreicht hat. Die chemischen und physikalischen Kenndaten der Reaktionsprodukte sind in der folgenden Tabelle zusammengefaßt:

| Beispiel | Polyisocyanat | Blockierungsmittel | NCO-Gehalt Gew.-% | | Polyisocyanat monomer | Viskosität mPa·s bei °C | | | | |
|---|---|---|---|---|---|---|---|---|---|---|
| DI | | | frei | gesamt | Gew.-% | 25 | 30 | 40 | 50 | 70 |
| 1 | IPDI | Caprolactam | 12,4 | 24,85 | 15,8 | 381 000 | 142 000 | 27 800 | 6 650 | 690 |
| 2 | IPDI | MEK-oxim | 12,4 | 26,0 | 15,3 | 27 000 | 14 750 | 3 390 | 1 100 | 195 |
| 3 | HDI | Caprolactam | 14,7 | 29,75 | 14,5 | 90 | 70 | 60 | 40 | < 30 |
| 4 | HDI | MEK-oxim | 16,4 | 31,5 | 14,5 | 75 | 55 | 35 | 30 | < 30 |
| 5 | DI 51 | Caprolactam | 14,5 | 29,0 | 14,3 | 190 | 140 | 70 | 40 | < 30 |
| 6 | DI 51 | MEK-oxim | 16,2 | 31,7 | 17,7 | 110 | 85 | 45 | 30 | < 30 |
| 7 | HMDI | Caprolactam | 10,9 | 21,9 | 17,0 | 880 000 | 300 000 | 60 000 | 9 000 | 1 100 |

D II Vergleichsbeispiele (Polyisocyanat-Harnstoff-Addukte)

Gemäß der allgemeinen Herstellungsvorschrift B für die blockierten Polyisocyanat-Harnstoff-Addukte wurden die partiell blockierten Polyisocyanate gemäß D I mit der Aminkomponente zur Reaktion gebracht. In der folgenden Tabelle sind die chemischen und physikalischen Kenndaten zusammengefaßt:

| Beispiel D II | Polyisocyanat gem. D I | Aminkomponente | NCO-Gehalt in Gew.-% | | Schmelzbereich in °C | Glasumwandlungstemperatur (DTA) °C |
|---|---|---|---|---|---|---|
| | | | latent | frei | | |
| 1 | 1 | 4.4'-Diaminodicyclohexylmethan | 9,4 | 0,2 | 158-160 | 19-110 |
| 2 | 3 | " | 10,6 | 0,1 | 102-104 | 17- 38 |
| 3 | 5 | " | 10,8 | 0 | 90- 92 | 14- 34 |
| 4 | 7 | " | 8,5 | 0 | 160-162 | 26- 85 |
| 5 | 1 | IPD | 9,9 | 0 | 141-145 | 20- 83 |

In allen Beispielen trat bereits während der Aminzugabe mehr oder minder starke Unverträglichkeit (Klumpenbildung) auf, so daß die Reaktion zwischen NCO- und Aminogruppen nicht einwandfrei ablief. Die Polyisocyanat-Harnstoff-Addukte wiesen noch eine schwache Basizität auf.

D III Vergleichsbeispiele (Polyisocyanat-Harnstoff-Addukte in lacküblichen Lösungsmitteln)

Werden die partiell blockierten Polyisocyanate gemäß D I gemäß der allgemeinen Herstellungsvorschrift C mit der Aminkomponente zur Reaktion gebracht, so fallen die Polyisocyanat-Harnstoff-Addukte bereits während der Aminzugabe aus und können für die Herstellung von lösemittelhaltigen Lacksystemen nicht verwendet werden.

**Patentansprüche**

1. Verfahren zur Herstellung von blockierten harnstoffgruppenhaltigen Polyisocyanaten aus halbblockierten Polyisocyanaten und Polyaminen,
dadurch gekennzeichnet,
daß man ein mittels Dünnschichtdestillation weitgehend von unblockiertem, monomerem Polyisocyanat befreites halbblockiertes Polyisocyanat mit primären und/oder sekundären Polyaminen im Isocyanat-/Aminogruppen-Verhältnis von 1 bis 1,3 : 1, bevorzugt 1 : 1 bei einer Temperatur im Bereich von RT bis 80 °C umsetzt, wobei das zur Dünnschichtdestillation eingesetzte Polyisocyanat einen Siedepunkt im Vakuum unterhalb der Deblockierungstemperatur des eingesetzten Blockierungsmittels besitzt, das halbblockierte Polyisocyanat durch Zugabe von 1 Mol Blockierungsmittel zu 5 bis 20 Mol Polyisocyanat erhalten wird und das halbblockierte Polyisocyanat nach der Dünnschichtdestillation einen Gehalt an monomerem, unblockiertem Polyisocyanat bis 6,5 Gew.-%, vorzugsweise bis 3,5 Gew.-% und insbesondere- bis 2,5 Gew.-%, aufweist.

2. Verfahren nach Anspruch 1,
dadurch gekennzeichnet,
daß als Polyamine primäre und/oder sekundäre aliphatische, aromatische, (cyclo)aliphatische und heterocyclische Polyamine oder deren Gemische gemäß den allgemeinen Formeln I und II eingesetzt werden:

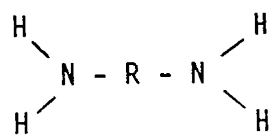

I

worin
R = $-(CH_2)_{2-36}$, geradkettig oder alkylverzweigt,

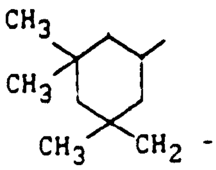 , 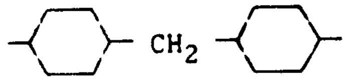 ,

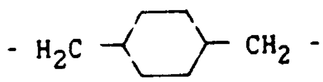 , 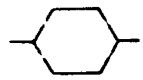 , 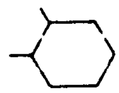 ,

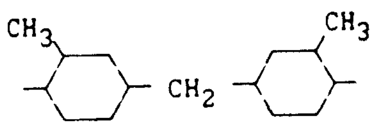 , 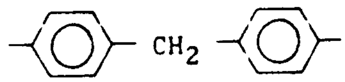 , 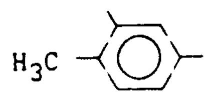 ,

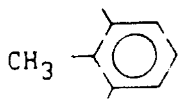 .

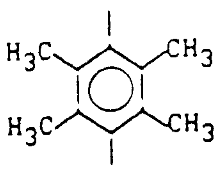

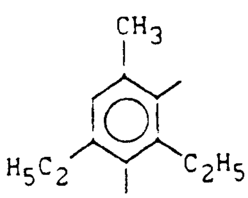 , 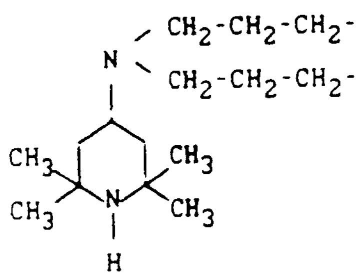

und

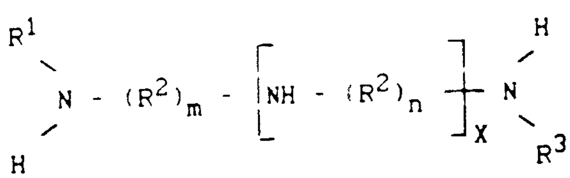 II

worin

| | | |
|---|---|---|
| $R^1$ | = | gleich oder verschieden von $R^3$ = H, alkyl-, (cyclo)-alkyl-, aryl-, aralkyl-, ggf. alkylverzweigt, |
| $R^2$ | = | $CH_2$-, ggf. alkylverzweigt, |
| m | = | 2 bis 8 |
| X | = | 0 bis 6 |
| n | = | 2 bis 8 |

ist.

3. Verfahren nach Anspruch 2,
dadurch gekennzeichnet,
daß bevorzugte Polyamine die Diamine Dodecamethylendiamin-1,12, 3,3'-Dimethyl-4,4'-diaminodicyclohexylmethan, Tetraethylenpentamin, Pentaethylenhexamin oder 4,4'-Diaminophenylmethan sind.

4. Verfahren nach Anspruch 2,
dadurch gekennzeichnet,
daß ganz besonders bevorzugte Diamine 4,4'-Diaminodicyclohexylmethan, Bis-(1,4-aminomethyl)-cyclohexan sowie Isophorondiamin, 2,2,4(2,4,4)-Trimethylhexamethylendiamin-1,6 und deren Gemische sind.

5. Verfahren nach Anspruch 1,
dadurch gekennzeichnet,
daß als bevorzugte Polyisocyanate aliphatische und/oder (cyclo)-aliphatische Diisocyanate eingesetzt werden.

6. Verfahren nach Anspruch 5,
dadurch gekennzeichnet,
daß als bevorzugte Diisocyanate Hexamethylendiisocyanat-1,6, 2-Methylpentandiisocyanat-1,5 und 2,2,4(2,4,4)-Trimethylhexamethylendiisocyanat-1,6 eingesetzt werden.

7. Verfahren nach Anspruch 5,
dadurch gekennzeichnet,
daß als besonders bevorzugtes Diisocyanat 3-Isocyanatomethyl-3,5,5-trimethylcyclohexylisocyanat eingesetzt wird.

8. Verfahren nach den Ansprüchen 1 bis 7,
dadurch gekennzeichnet,
daß die Reaktionskomponenten im Lösungsmittel eingesetzt werden und nach erfolgter Reaktion das Lösungsmittel verbleibt oder entfernt wird.

9. Verfahren nach den Ansprüchen 1 bis 8,
dadurch gekennzeichnet,
daß das Polyamin in Substanz oder im Lösungsmittel dem partiell blockierten Polyisocyanat zugesetzt wird.

10. Verfahren nach den Ansprüchen 1 und 9,
dadurch gekennzeichnet,
daß als Lösungsmittel die lacküblichen Lösungsmittel eingesetzt werden.

11. Verfahren nach den Ansprüchen 1 und 9,
dadurch gekennzeichnet,
daß als Lösungsmittel Toluol, Cyclohexan und Spezialbenzin mit = 1 Vol.-% Aromaten eingesetzt werden.

12. Verfahren nach den Ansprüchen 1 bis 11,
dadurch gekennzeichnet,
daß der NCO-Gehalt der Polyisocyanat-Harnstoff-Addukte 4 bis 20 Gew.-%, bevorzugt 6 bis 15 Gew.-%, beträgt.

13. Verfahren nach den Ansprüchen 1 bis 12,
dadurch gekennzeichnet,
daß der freie NCO-Gehalt der Polyisocyanat-Harnstoffaddukte von 0,7 bis 5 Gew.-% beträgt.

14. Verwendung der blockierten Polyisocyanat-Harnstoffaddukte, hergestellt gemäß den Ansprüchen 1 bis 13, in Polyurethan-Kunststoffen.

## Claims

**1.** A process for the production of blocked polyisocyanates containing urea groups consisting of semi-blocked polyisocyanates and polyamines,
**characterised in that** a semi-blocked polyisocyanate, which is largely released from unblocked, monomer polyisocyanate by means of thin-layer distillation, having primary and/or secondary polyamines in the isocyanate/amino group ratio of 1 to 1.3 : 1, and preferably 1 : 1, is transformed at a temperature in the range from RT to 80 °C, the polyisocyanate used for the thin-layer distillation having a boiling point in vacuum below the deblocking temperature of the blocking agent used, the semi-blocked polyisocyanate being obtained by the addition of 1 mole blocking agent to 5 to 20 moles polyisocyanate and after the thin-layer distillation the semi-blocked polyisocyanate having a content of monomer, unblocked polyisocyanate of up to 6.5 % by weight, preferably up to 3.5 % by weight and in particular up to 2.5 % by weight.

**2.** A process according to Claim 1,
**characterised in that** primary and/or secondary aliphatic, aromatic, (cyclo)aliphatic and heterocylic polyamines or mixtures thereof in accordance with the general formulae I and II are used as polyamines:

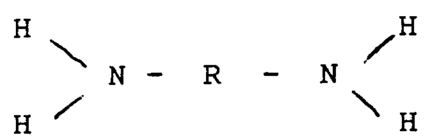

I

in which
R = $-(CH_2)_{2-36}$, straight-chain or alkyl-branched,

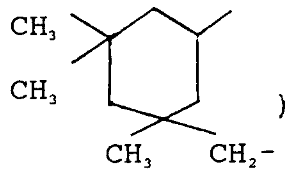,
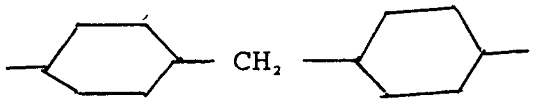,

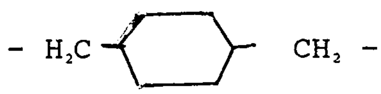,
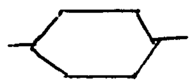,
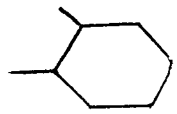,

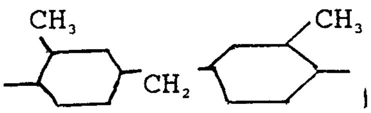,
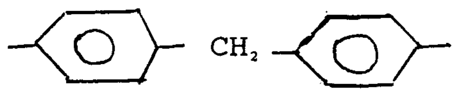,
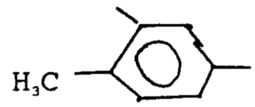,

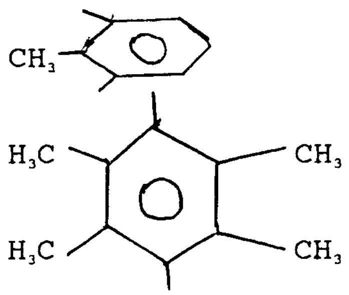

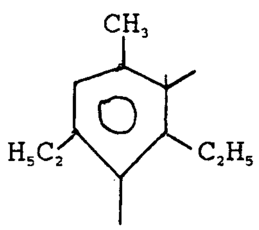,
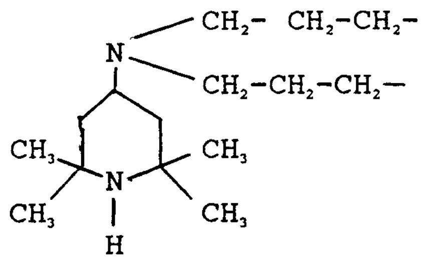

and

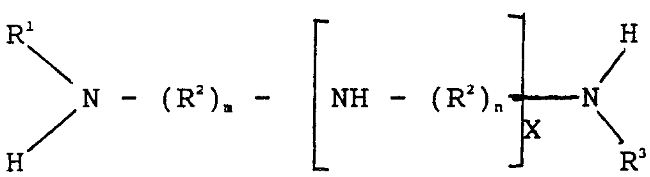 II

in which

R1 = the same as or different to $R^3$ = H, alkyl-, (cyclo)alkyl-, aryl-, aralkyl-, and if necessary, alkyl-branched,

$R^2$ = $CH_2$-, alkyl-branched if necessary,

m = 2 to 8

X = 0 to 6

n = 2 to 8.

**3.** A process according to Claim 2, **characterised in that** the diamines dodecamethylenediamine-1,12, 3,3'-dimethyl-4,4'-diaminodicyclohexylmethane, tetraethylene pentamine, pentaethylene hexamine or 4,4'-diaminophenylmethane are preferred polyamines.

4. A process according to Claim 2, **characterised in that** 4,4'-diaminodicyclohexylmethane, bis-(1,4-aminomethyl)-cyclohexane and also isophorone diamine, 2,2,4(2,4,4)-trimethylhexamethylene diamine-1,6 and mixtures thereof are particularly preferred diamines.

5. A process according to Claim 1, **characterised in that** aliphatic and/or (cyclo)-aliphatic di-isocyanates are used as preferred polyisocyanates.

6. A process according to Claim 5, **characterised in that** hexamethylene diisocyanate-1,6, 2-methylpentane diioscyanate-1,5 and 2,2,4-(2,4,4)-trimethylhexamethylene diisocyanate-1,6 are used as preferred diisocyanates.

7. A process according to Claim 5, **characterised in that** 3-isocyanatomethyl-3,5,5-trimethylcyclohexyl isocyanate is used as a particularly preferred diisocyanate.

8. A process according to Claims 1 to 7, **characterised in that** the reaction constituents are used in the solvent and after the reaction is complete the solvent remains or is removed.

9. A process according to Claims 1 to 8, **characterised in that** the polyamine is added to the partially blocked polyisocyanate in bulk or in the solvent.

10. A process according to Claims 1 and 9, **characterised in that** the usual solvents for lacquers are used as solvents.

11. A process according to Claims 1 and 9, **characterised in that** toluene, cyclohexane and special-grade petroleum spirit with aromatic compounds = 1 % by volume are used as solvents.

12. A process according to Claims 1 to 11, **characterised in that** the NCO content of the polyisocyanate-urea addition compounds is 4 to 20 % by weight, preferably 6 to 15 % by weight.

13. A process according to Claims 1 to 12, **characterised in that** the free NCO content of the polyisocyanate-urea addition compounds is from 0.7 to 5 % by weight.

14. Use of the blocked polyisocyanate urea-addition compounds, produced in accordance with Claims 1 to 13, in polyurethane plastics.

**Revendications**

1. Procédé d'obtention de polyisocyanates renfermant des groupes urée, bloqués, à partir de polyisocyanates à demi bloqués et de polyamines, caractérisé en ce que l'on fait réagir un polyisocyanate à demi bloqué, débarrassé de polyisocyanate monomère non bloqué, avec des polyamines primaires et/ou secondaires dans un rapport isocyanate/groupes aminés de 1 à 1,3 : 1 de préférence 1 : 1 à une température dans la plage allant de la température ordinaire à 80 °C, procédé dans lequel le polyisocyanate mis en oeuvre en vue d'une distillation en couche mince possède un point d'ébullition sous vide, en dessous de la température de déblocage de l'agent de blocage mis en jeu, que le polyisocyanate à demi bloqué est obtenu par addition de 1 mol d'agent de blocage pour 5 à 20 moi de polyisocyanate et le polyisocyanate à demi bloqué après distillation en couche mince possède une teneur en polyisocyanate monomérique, non bloqué allant jusqu'à 6,5 % en poids, de préférence jusqu'à 3,5 % en poids et en particulier jusqu'à 2,5 % en poids.

2. Procédé selon la revendication 1, caractérisé en ce que comme polyamine on met en jeu des polyamines primaires et/ou secondaires, aliphatiques, aromatiques, (cyclo)aliphatiques et hétérocycliques ou leurs mélanges conformément aux formules générales I et II

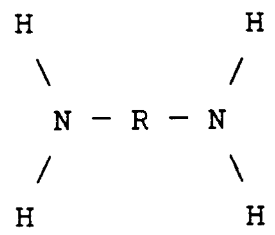

I

dans laquelle R est $(CH_2)$-$_{2-36}$, un alcoyle à chaîne droite ou ramifiée,

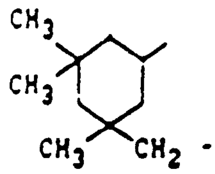 , 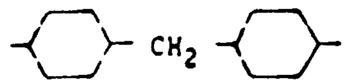 ,

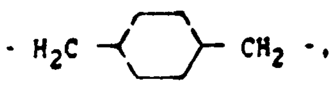 , 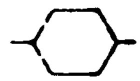 , 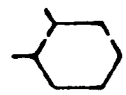 ,

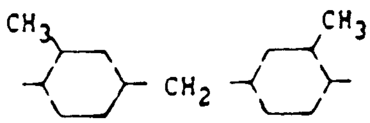 , 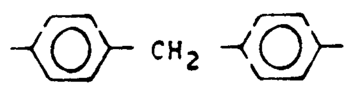 , 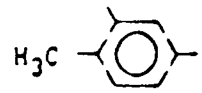 ,

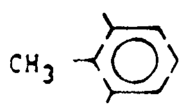 .

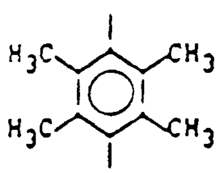

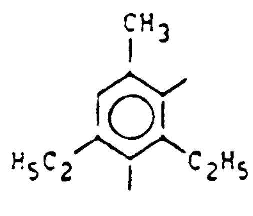 , 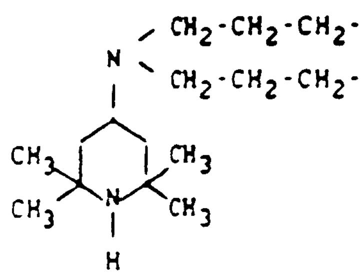

et

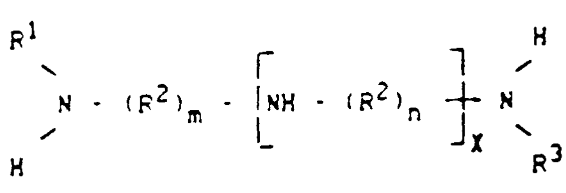 II

dans laquelle

| | |
|---|---|
| $R^1$ | est, égal ou différent de $R^3$ = H, alcoyl, (cyclo)alcoyl, aryle, aralcoyle, alcoyle éventuellement ramifié, |
| $R^2$ = | $CH_2$-, alcoyle éventuellement ramifié, |
| m = | 2 à 8 |
| x = | 0 à 6 |
| n = | 2 à 8 |

**3.** Procédé selon la revendication 2, caractérisé en ce que les polyamines préférées sont les diamines dodécalméthylène diamine-1,12, le 3,3'-diméthyle-4-4'-diamino-dicyclohexylméthane, la tétraéthylène

pentamine, la pentaéthylènehexamine ou le 4,4-diaminophenylméthane.

4. Procédé selon la revendication 2, caractérisé en ce que les diamines particulièrement préférées sont le 4,4'-diaminodicyclohexylméthane, le bis-(1,4-aminométhyl) cyclohexane ainsi que l'isophoronediamine, la 2,2,4(2,4,4)- triméthylhexaméthylène diamine-1,6 et leurs mélanges.

5. Procédé selon la revendication 1, caractérisé en ce que comme polyisocyanates préférés, des diisocyanates aliphatiques et/ou (cyclo)aliphatiques sont mis en oeuvre.

6. Procédé selon la revendication 5, caractérisé en ce que comme diisocyanates préférés, on met en oeuvre l'hexaméthylène diisocyanate-1,6, le 2-méthylpentanediisocyanate-1,5 et le 2,2,4(2,4,4)-triméthylhexaméthylène diisocyanate-1,6.

7. Procédé selon la revendication 5, caractérisé en ce que comme diisocyanate particulièrement préféré, on met en oeuvre le 3-isocyanatométhyl-3,5,5-triméthylcyclohexylisocyanate.

8. Procédé selon les revendications 1 à 7, caractérisé en ce que les composants de la réaction sont mise en oeuvre dans un solvant et après achèvement de la réaction, le solvant demeure ou est chassé.

9. Procédé selon les revendications 1 à 8, caractérisé en ce que la polyamine est ajoutée en substance ou dans un solvant, au polyisocyanate partiellement bloqué.

10. Procédé selon les revendications 1 et 9, caractérisé en ce que comme solvant on met en oeuvre des solvants habituels des laques.

11. Procédé selon les revendications 1 et 9, caractérisé en ce que comme solvant le toluène, le cyclohexane et l'essence spéciale avec 1 % en volume d'aromatiques, sont mis en oeuvre.

12. Procédé selon les revendications 1 à 11, caractérisé en ce que la teneur en NCO du produit d'addition polyisocyanate/urée s'élève à 4 à 20 % en poids, de préférence de 6 à 15 % en poids.

13. Procédé selon les revendications 1 à 12, caractérisé en ce que la teneur en NCO libres des produits d'addition polyisocyanate/urée s'élève de 0,7 à 5 % en poids.

14. Utilisation des produits d'addition polyisocyanate/urée obtenu selon les revendications 1 à 13 dans les matières plastiques à base de polyuréthane.